# EUROPEAN PATENT APPLICATION

(11) **EP 3 345 924 A1**
(43) Date of publication of application: **11.07.2018**
(21) Application number: 17150813.8
(22) Date of filing: 10.01.2017
(51) Int. Cl.: C07K 16/28, A61P 9/10, A61K 39/395

(54) **USE OF CD47 ANTIBODIES**

(71) Applicant: Universität Duisburg-Essen, 45141 Essen (DE)
(72) Inventor: Rassaf, Tienush, 45133 Essen (DE); Totzeck, Matthias, 45888 Gelsenkirchen (DE)
(74) Representative: Gramm, Lins & Partner Patent- und Rechtsanwälte PartGmbB

(57) **Abstract**

The present invention relates to CD47 antibodies or fragments thereof binding specifically to the CD47 immunogen for use in preventing or treating tissue injury in states of ischemia or reperfusion in an object whereby the CD47 antibodies or fragments thereof are introduced intravascularly at the or near by the place of ischemia or reperfusion.

SEQUENCE LISTING

<110>  Universitaet Duisburg-Essen

<120>  Use of CD47 antibodies

<130>  5951-001 EP-1

<160>  82

<170>  PatentIn version 3.5

<210>  1
<211>  879
<212>  DNA
<213>  homo sapiens


<220>
<221>  CDS
<222>  (1)..(879)

<400>  1
atg tgg ccc ctg gta gcg gcg ctg ttg ctg ggc tcg gcg tgc tgc gga      48
Met Trp Pro Leu Val Ala Ala Leu Leu Leu Gly Ser Ala Cys Cys Gly
1               5                   10                  15

tca gct cag cta cta ttt aat aaa aca aaa tct gta gaa ttc acg ttt      96
Ser Ala Gln Leu Leu Phe Asn Lys Thr Lys Ser Val Glu Phe Thr Phe
            20                  25                  30

tgt aat gac act gtc gtc att cca tgc ttt gtt act aat atg gag gca      144
Cys Asn Asp Thr Val Val Ile Pro Cys Phe Val Thr Asn Met Glu Ala
        35                  40                  45

caa aac act act gaa gta tac gta aag tgg aaa ttt aaa gga aga gat      192
Gln Asn Thr Thr Glu Val Tyr Val Lys Trp Lys Phe Lys Gly Arg Asp
    50                  55                  60

att tac acc ttt gat gga gct cta aac aag tcc act gtc ccc act gac      240
Ile Tyr Thr Phe Asp Gly Ala Leu Asn Lys Ser Thr Val Pro Thr Asp
65                  70                  75                  80

ttt agt agt gca aaa att gaa gtc tca caa tta cta aaa gga gat gcc      288
Phe Ser Ser Ala Lys Ile Glu Val Ser Gln Leu Leu Lys Gly Asp Ala
                85                  90                  95

tct ttg aag atg gat aag agt gat gct gtc tca cac aca gga aac tac      336
Ser Leu Lys Met Asp Lys Ser Asp Ala Val Ser His Thr Gly Asn Tyr
            100                 105                 110

act tgt gaa gta aca gaa tta acc aga gaa ggt gaa acg atc atc gag      384
Thr Cys Glu Val Thr Glu Leu Thr Arg Glu Gly Glu Thr Ile Ile Glu
        115                 120                 125

cta aaa tat cgt gtt gtt tca tgg ttt tct cca aat gaa aat att ctt      432
Leu Lys Tyr Arg Val Val Ser Trp Phe Ser Pro Asn Glu Asn Ile Leu
    130                 135                 140

att gtt att ttc cca att ttt gct ata ctc ctg ttc tgg gga cag ttt      480
Ile Val Ile Phe Pro Ile Phe Ala Ile Leu Leu Phe Trp Gly Gln Phe
145                 150                 155                 160

ggt att aaa aca ctt aaa tat aga tcc ggt ggt atg gat gag aaa aca     528
Gly Ile Lys Thr Leu Lys Tyr Arg Ser Gly Gly Met Asp Glu Lys Thr
                165                 170                 175

att gct tta ctt gtt gct gga cta gtg atc act gtc att gtc att gtt     576
Ile Ala Leu Leu Val Ala Gly Leu Val Ile Thr Val Ile Val Ile Val
            180                 185                 190

gga gcc att ctt ttc gtc cca ggt gaa tat tca tta aag aat gct act     624
Gly Ala Ile Leu Phe Val Pro Gly Glu Tyr Ser Leu Lys Asn Ala Thr
        195                 200                 205

ggc ctt ggt tta att gtg act tct aca ggg ata tta ata tta ctt cac     672
Gly Leu Gly Leu Ile Val Thr Ser Thr Gly Ile Leu Ile Leu Leu His
    210                 215                 220

tac tat gtg ttt agt aca gcg att gga tta acc tcc ttc gtc att gcc     720
Tyr Tyr Val Phe Ser Thr Ala Ile Gly Leu Thr Ser Phe Val Ile Ala
225                 230                 235                 240

ata ttg gtt att cag gtg ata gcc tat atc ctc gct gtg gtt gga ctg     768
Ile Leu Val Ile Gln Val Ile Ala Tyr Ile Leu Ala Val Val Gly Leu
                245                 250                 255

agt ctc tgt att gcg gcg tgt ata cca atg cat ggc cct ctt ctg att     816
Ser Leu Cys Ile Ala Ala Cys Ile Pro Met His Gly Pro Leu Leu Ile
            260                 265                 270

tca ggt ttg agt atc tta gct cta gca caa tta ctt gga cta gtt tat     864
Ser Gly Leu Ser Ile Leu Ala Leu Ala Gln Leu Leu Gly Leu Val Tyr
        275                 280                 285

atg aaa ttt gtg gaa                                                 879
Met Lys Phe Val Glu
    290


<210>  2
<211>  293
<212>  PRT
<213>  homo sapiens

<400>  2

Met Trp Pro Leu Val Ala Ala Leu Leu Leu Gly Ser Ala Cys Cys Gly
1               5                   10                  15


Ser Ala Gln Leu Leu Phe Asn Lys Thr Lys Ser Val Glu Phe Thr Phe
            20                  25                  30


Cys Asn Asp Thr Val Val Ile Pro Cys Phe Val Thr Asn Met Glu Ala
        35                  40                  45


Gln Asn Thr Thr Glu Val Tyr Val Lys Trp Lys Phe Lys Gly Arg Asp
    50                  55                  60


Ile Tyr Thr Phe Asp Gly Ala Leu Asn Lys Ser Thr Val Pro Thr Asp
65                  70                  75                  80

Phe Ser Ser Ala Lys Ile Glu Val Ser Gln Leu Leu Lys Gly Asp Ala
                85                  90                  95

Ser Leu Lys Met Asp Lys Ser Asp Ala Val Ser His Thr Gly Asn Tyr
            100                 105                 110

Thr Cys Glu Val Thr Glu Leu Thr Arg Glu Gly Glu Thr Ile Ile Glu
        115                 120                 125

Leu Lys Tyr Arg Val Val Ser Trp Phe Ser Pro Asn Glu Asn Ile Leu
    130                 135                 140

Ile Val Ile Phe Pro Ile Phe Ala Ile Leu Leu Phe Trp Gly Gln Phe
145                 150                 155                 160

Gly Ile Lys Thr Leu Lys Tyr Arg Ser Gly Gly Met Asp Glu Lys Thr
                165                 170                 175

Ile Ala Leu Leu Val Ala Gly Leu Val Ile Thr Val Ile Val Ile Val
            180                 185                 190

Gly Ala Ile Leu Phe Val Pro Gly Glu Tyr Ser Leu Lys Asn Ala Thr
        195                 200                 205

Gly Leu Gly Leu Ile Val Thr Ser Thr Gly Ile Leu Ile Leu Leu His
    210                 215                 220

Tyr Tyr Val Phe Ser Thr Ala Ile Gly Leu Thr Ser Phe Val Ile Ala
225                 230                 235                 240

Ile Leu Val Ile Gln Val Ile Ala Tyr Ile Leu Ala Val Val Gly Leu
                245                 250                 255

Ser Leu Cys Ile Ala Ala Cys Ile Pro Met His Gly Pro Leu Leu Ile
            260                 265                 270

Ser Gly Leu Ser Ile Leu Ala Leu Ala Gln Leu Leu Gly Leu Val Tyr
        275                 280                 285

Met Lys Phe Val Glu
    290

<210>  3
<211>  5
<212>  PRT
<213>  artificial

<220>

<223>  peptide

<400>  3

Gly Tyr Gly Met Ser
1               5


<210>  4
<211>  17
<212>  PRT
<213>  artificial

<220>
<223>  peptide

<400>  4

Thr Ile Thr Ser Gly Gly Thr Tyr Thr Tyr Tyr Pro Asp Ser Val Lys
1               5                   10                  15


Gly




<210>  5
<211>  9
<212>  PRT
<213>  artificial

<220>
<223>  peptide

<400>  5

Ser Leu Ala Gly Asn Ala Met Asp Tyr
1               5


<210>  6
<211>  5
<212>  PRT
<213>  artificial

<220>
<223>  peptide

<400>  6

Asn Tyr Asn Met His
1               5


<210>  7
<211>  17
<212>  PRT
<213>  artificial

<220>
<223>  peptide

<400>  7

Thr Ile Tyr Pro Gly Asn Asp Asp Thr Ser Tyr Asn Gln Lys Phe Lys
1               5                   10                  15

Asp

<210>  8
<211>  8
<212>  PRT
<213>  artificial

<220>
<223>  peptide

<400>  8

Gly Gly Tyr Arg Ala Met Asp Tyr
1               5

<210>  9
<211>  5
<212>  PRT
<213>  artificial

<220>
<223>  peptide

<400>  9

Thr Tyr Val Val His
1               5

<210>  10
<211>  17
<212>  PRT
<213>  artificial

<220>
<223>  peptide

<400>  10

Tyr Ile Asn Pro Tyr Asn Asp Gly Thr Lys Tyr Asn Glu Lys Phe Lys
1               5                   10                  15

Gly

<210>  11
<211>  11
<212>  PRT
<213>  artificial

<220>
<223>  peptide

<400>  11

Gly Tyr Tyr Arg Tyr Gly Tyr Thr Met Asp Tyr
1               5                   10


<210>  12
<211>  11
<212>  PRT
<213>  artificial

<220>
<223>  peptide

<400>  12

Arg Ala Ser Gln Thr Ile Ser Asp Tyr Leu His
1               5                   10


<210>  13
<211>  7
<212>  PRT
<213>  artificial

<220>
<223>  peptide

<400>  13

Phe Ala Ser Gln Ser Ile Ser
1               5


<210>  14
<211>  9
<212>  PRT
<213>  artificial

<220>
<223>  peptide

<400>  14

Gln Asn Gly His Gly Phe Pro Arg Thr
1               5


<210>  15
<211>  16
<212>  PRT
<213>  artificial

<220>
<223>  peptide

<400>  15

Arg Ser Ser Gln Ser Ile Val Tyr Ser Asn Gly Asn Thr Tyr Leu Gly
1               5                   10                  15


<210>  16

<211>  7
<212>  PRT
<213>  artificial

<220>
<223>  peptide

<400>  16

Lys Val Ser Asn Arg Phe Ser
1               5


<210>  17
<211>  9
<212>  PRT
<213>  artificial

<220>
<223>  peptide

<400>  17

Phe Gln Gly Ser His Val Pro Tyr Thr
1               5


<210>  18
<211>  11
<212>  PRT
<213>  artificial

<220>
<223>  peptide

<400>  18

Arg Ala Ser Gln Asn Phe Ser Asp Tyr Leu His
1               5                   10


<210>  19
<211>  7
<212>  PRT
<213>  artificial

<220>
<223>  peptide

<400>  19

Tyr Val Ser His Ser Ile Ser
1               5


<210>  20
<211>  9
<212>  PRT
<213>  artificial

<220>
<223>  peptide

<400>  20

Gln Asn Gly His Ser Phe Pro Pro Thr
1               5


<210>  21
<211>  106
<212>  PRT
<213>  artificial

<220>
<223>  peptide

<400>  21

Asp Val Val Met Thr Gln Thr Pro Leu Ser Leu Ser Val Ser Leu Gly
1               5                   10                  15


Asp Gln Ala Ser Ile Ser Cys Arg Ser Ser Gln Ser Leu Val His Ser
            20                  25                  30


Asn Gly Asn Thr Tyr Leu His Trp Tyr Leu Gln Lys Pro Gly Gln Ser
        35                  40                  45


Pro Lys Leu Leu Ile Tyr Lys Val Ser Tyr Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Leu Gly Val Tyr Phe Cys Ser Gln Asn
                85                  90                  95


Thr His Val Pro Arg Thr Phe Gly Gln Gly
            100                 105


<210>  22
<211>  106
<212>  PRT
<213>  artificial

<220>
<223>  peptie

<400>  22

Asp Ile Val Met Thr Gln Thr Pro Leu Ser Leu Pro Val Thr Pro Gly
1               5                   10                  15


Glu Pro Ala Ser Ile Ser Cys Arg Ser Ser Gln Ser Leu Val His Ser
            20                  25                  30


Asn Gly Asn Thr Tyr Leu His Trp Tyr Gln Gln Lys Pro Gly Lys Ala

35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  23
<211>  106
<212>  PRT
<213>  artificial

<220>
<223>  peptide

<400>  23

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Ser Arg Phe Ser Gly Ser Gly Ser Gly Thr Glu Phe Thr Leu Thr Ile
65                  70                  75                  80

Ser Ser Leu Gln Pro Asp Asp Phe Ala Thr Tyr Tyr Cys Ser Gln Asn
                85                  90                  95

Thr His Val Pro Arg Thr Phe Gly Gln Gly
            100                 105

<210>  24
<211>  106
<212>  PRT
<213>  artificial

<220>

<223>  peptide

<400>  24

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  25
<211>  106
<212>  PRT
<213>  artificial

<220>
<223>  peptide

<400>  25

Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Tyr Arg Phe Ser Gly Ile Pro
    50                  55                  60

Ala Arg Phe Ser Gly Ser Gly Ser Gly Thr Glu Phe Thr Leu Thr Ile
65                  70                  75                  80

Ser Ser Leu Gln Ser Glu Asp Phe Ala Val Tyr Tyr Cys Ser Gln Asn

85                  90                  95

Thr His Val Pro Arg Thr Phe Gly Gln Gly
            100                 105

<210>  26
<211>  106
<212>  PRT
<213>  artificial

<220>
<223>  peptide

<400>  26

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  27
<211>  106
<212>  PRT
<213>  artificial

<220>
<223>  peptide

<400>  27

Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Tyr Arg Phe Ser Gly Ile Pro
    50                  55                  60

Ala Arg Phe Ser Gly Ser Gly Ser Gly Thr Glu Phe Thr Leu Thr Ile
65                  70                  75                  80

Ser Ser Leu Gln Ser Glu Asp Phe Ala Val Tyr Tyr Cys Ser Gln Asn
                85                  90                  95

Thr His Val Pro Arg Thr Phe Gly Gln Gly
            100                 105

<210>  28
<211>  106
<212>  PRT
<213>  artificial

<220>
<223>  peptide

<400>  28

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  29
<211>  106
<212>  PRT
<213>  artificial

<220>
<223>  peptide

<400>  29

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105


<210>  30
<211>  106
<212>  PRT
<213>  artificial

<220>
<223>  peptide

<400>  30

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  31
<211>  106
<212>  PRT
<213>  artificial

<220>
<223>  peptide

<400>  31

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  32
<211>  106
<212>  PRT
<213>  artificial

<220>
<223>  peptide

<400>  32

Glu Ile Val Leu Thr Gln Ser Pro Ala Thr Leu Ser Val Ser Pro Gly
1               5                   10                  15

Glu Arg Ala Thr Leu Ser Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Ser Arg Phe Ser Gly Ser Gly Ser Gly Thr Asp Phe Thr Phe Thr Ile
65                  70                  75                  80

Ser Ser Leu Glu Ala Glu Asp Ala Ala Thr Tyr Tyr Cys Ser Gln Asn
                85                  90                  95

Thr His Val Pro Arg Thr Phe Gly Gln Gly
            100                 105

<210>  33
<211>  106
<212>  PRT
<213>  artificial

<220>
<223>  peptide

<400>  33

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  34
<211>  106
<212>  PRT
<213>  artificial

<220>
<223>  peptide

<400>  34

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105


<210>  35
<211>  106
<212>  PRT
<213>  artificial

<220>
<223>  peptide

<400>  35

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  36
<211>  106
<212>  PRT
<213>  artificial

<220>
<223>  peptide

<400>  36

Ala Ile Gln Leu Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Ser Arg Phe Ser Gly Ser Gly Ser Gly Thr Glu Phe Thr Leu Thr Ile
65                  70                  75                  80

Ser Ser Leu Gln Pro Asp Asp Phe Ala Thr Tyr Tyr Cys Ser Gln Asn
                85                  90                  95

Thr His Val Pro Arg Thr Phe Gly Gln Gly
            100                 105

<210>  37
<211>  106
<212>  PRT
<213>  artificial

<220>
<223>  peptide

<400>  37

Ala Ile Gln Leu Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  38
<211>  106
<212>  PRT
<213>  artificial

<220>
<223>  peptide

<400>  38

Ala Ile Gln Leu Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  39
<211>  106
<212>  PRT

<213>  artificial

<220>
<223>  peptide

<400>  39

Glu Ile Val Leu Thr Gln Ser Pro Ala Thr Leu Ser Val Ser Pro Gly
1               5                   10                  15

Glu Arg Ala Thr Leu Ser Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Ser Arg Phe Ser Gly Ser Gly Ser Gly Thr Asp Phe Thr Phe Thr Ile
65                  70                  75                  80

Ser Ser Leu Glu Ala Glu Asp Ala Ala Thr Tyr Tyr Cys Ser Gln Asn
                85                  90                  95

Thr His Val Pro Arg Thr Phe Gly Gln Gly
            100                 105

<210>  40
<211>  106
<212>  PRT
<213>  artificial

<220>
<223>  peptide

<400>  40

Asp Val Val Met Thr Gln Ser Pro Leu Ser Leu Pro Val Thr Leu Gly
1               5                   10                  15

Gln Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  41
<211>  106
<212>  PRT
<213>  artificial

<220>
<223>  peptide

<400>  41

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  42
<211>  106
<212>  PRT
<213>  artificial

<220>
<223>  peptide

<400>  42

Ala Ile Gln Leu Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser

20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105

<210>  43
<211>  106
<212>  PRT
<213>  artificial

<220>
<223>  peptide

<400>  43

Glu Ile Val Leu Thr Gln Ser Pro Ala Thr Leu Ser Val Ser Pro Gly
1               5                   10                  15

Glu Arg Ala Thr Leu Ser Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Ser Arg Phe Ser Gly Ser Gly Ser Gly Thr Asp Phe Thr Phe Thr Ile
65                  70                  75                  80

Ser Ser Leu Glu Ala Glu Asp Ala Ala Thr Tyr Tyr Cys Ser Gln Asn
                85                  90                  95

Thr His Val Pro Arg Thr Phe Gly Gln Gly
            100                 105

<210>  44
<211>  106

<212>  PRT
<213>  artificial

<220>
<223>  peptide

<400>  44

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Tyr Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Arg Thr Phe Gly Gln Gly
            100                 105


<210>  45
<211>  106
<212>  PRT
<213>  artificial

<220>
<223>  peptide

<400>  45

Ala Ile Gln Leu Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Tyr Leu His Trp Tyr Gln Gln Lys Pro Gly Gln Ala
        35                  40                  45

Pro Arg Leu Leu Ile Tyr Lys Val Ser Tyr Arg Phe Ser Gly Val Pro
    50                  55                  60

Ser Arg Phe Ser Gly Ser Gly Ser Gly Thr Glu Phe Thr Leu Thr Ile

65                  70                  75                  80

Ser Ser Leu Gln Pro Asp Asp Phe Ala Thr Tyr Tyr Cys Ser Gln Asn
                85                  90                  95

Thr His Val Pro Arg Thr Phe Gly Gln Gly
            100                 105

<210>  46
<211>  113
<212>  PRT
<213>  artificial

<220>
<223>  peptide

<400>  46

Thr Lys Val Glu Ile Lys Arg Thr Val Ala Ala Pro Ser Val Phe Ile
1               5                   10                  15

Phe Pro Pro Ser Asp Glu Gln Leu Lys Ser Gly Thr Ala Ser Val Val
            20                  25                  30

Cys Leu Leu Asn Asn Phe Tyr Pro Arg Glu Ala Lys Val Gln Trp Lys
        35                  40                  45

Val Asp Asn Ala Leu Gln Ser Gly Asn Ser Gln Glu Ser Val Thr Glu
    50                  55                  60

Gln Asp Ser Lys Asp Ser Thr Tyr Ser Leu Ser Ser Thr Leu Thr Leu
65                  70                  75                  80

Ser Lys Ala Asp Tyr Glu Lys His Lys Val Tyr Ala Cys Glu Val Thr
                85                  90                  95

His Gln Gly Leu Ser Ser Pro Val Thr Lys Ser Phe Asn Arg Gly Glu
            100                 105                 110

Cys

<210>  47
<211>  109
<212>  PRT
<213>  artificial

<220>
<223>  peptide

<400>  47

Glu Val Gln Leu Gln Gln Phe Gly Ala Glu Leu Val Lys Pro Gly Ala
1               5                   10                  15

Ser Met Lys Leu Ser Cys Lys Ala Ser Gly Tyr Thr Phe Thr Asn Tyr
            20                  25                  30

Tyr Val Phe Trp Val Lys Gln Arg Pro Gly Gln Gly Leu Glu Trp Ile
        35                  40                  45

Gly Asp Ile Asn Pro Val Asn Gly Asp Thr Asn Phe Asn Glu Lys Phe
    50                  55                  60

Lys Asn Lys Ala Thr Leu Thr Val Asp Lys Ser Ser Thr Thr Thr Tyr
65                  70                  75                  80

Leu Gln Leu Ser Ser Leu Thr Ser Glu Asp Ser Ala Val Tyr Tyr Cys
                85                  90                  95

Thr Arg Gly Gly Tyr Thr Met Asp Tyr Trp Gly Gln Gly
            100                 105

<210>  48
<211>  109
<212>  PRT
<213>  artificial

<220>
<223>  peptide

<400>  48

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gly Tyr Thr Met Asp Tyr Trp Gly Gln Gly

100                 105

<210>  49
<211>  109
<212>  PRT
<213>  artificial

<220>
<223>  peptide

<400>  49

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gly Tyr Thr Met Asp Tyr Trp Gly Gln Gly
            100                 105

<210>  50
<211>  109
<212>  PRT
<213>  artificial

<220>
<223>  peptide

<400>  50

Glu Val Gln Leu Val Gln Ser Gly Ala Glu Val Lys Lys Pro Gly Glu
1               5                   10                  15

Ser Leu Arg Ile Ser Cys Lys Gly Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  51
<211>  109
<212>  PRT
<213>  artificial

<220>
<223>  peptide

<400>  51

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Pro Gly Lys Gly Leu Glu Trp Val
        35                  40                  45

Ser Asp Ile Asn Pro Val Asn Gly Asp Thr Asn Phe Asn Glu Lys Phe
    50                  55                  60

Lys Asn Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Gly Tyr Thr Met Asp Tyr Trp Gly Gln Gly
            100                 105

<210>  52
<211>  109
<212>  PRT
<213>  artificial

<220>
<223>  peptide

<400>  52

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Pro Gly Lys Gly Leu Glu Trp Val
        35                  40                  45

Ser Asp Ile Asn Pro Val Asn Gly Asp Thr Asn Phe Asn Glu Lys Phe
    50                  55                  60

Lys Asn Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Gly Tyr Thr Met Asp Tyr Trp Gly Gln Gly
            100                 105

<210>  53
<211>  109
<212>  PRT
<213>  artificial

<220>
<223>  peptide

<400>  53

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys
                85                  90                  95

Ala Arg Gly Gly Tyr Thr Met Asp Tyr Trp Gly Gln Gly
            100                 105

<210>  54
<211>  109
<212>  PRT
<213>  artificial

<220>
<223>  peptide

<400>  54

Gln Val Gln Leu Gln Glu Ser Gly Pro Gly Leu Val Lys Pro Gly Ala
1               5                   10                  15

Thr Val Lys Ile Ser Cys Lys Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  55
<211>  109
<212>  PRT
<213>  artificial

<220>
<223>  peptide

<400>  55

Gln Ile Thr Leu Lys Glu Ser Gly Pro Thr Leu Val Lys Pro Thr Gln
1               5                   10                  15

Thr Leu Thr Leu Thr Cys Thr Phe Ser Gly Tyr Thr Phe Thr Asn Tyr
            20                  25                  30

Tyr Val Phe Trp Ile Arg Gln Ser Pro Ser Arg Gly Leu Glu Trp Leu
        35                  40                  45

Gly Asp Ile Asn Pro Val Asn Gly Asp Thr Asn Phe Asn Glu Lys Phe
    50                  55                  60

Lys Asn Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Tyr Thr Met Asp Tyr Trp Gly Gln Gly
            100                 105

<210>  56
<211>  109
<212>  PRT
<213>  artificial

<220>
<223>  peptide

<400>  56

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys
                85                  90                  95

Ala Arg Gly Gly Tyr Thr Met Asp Tyr Trp Gly Gln Gly
            100                 105

<210>  57
<211>  109
<212>  PRT
<213>  artificial

<220>
<223>  peptide

<400>  57

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys
                85                  90                  95

Ala Arg Gly Gly Tyr Thr Met Asp Tyr Trp Gly Gln Gly
            100                 105

<210>  58
<211>  109
<212>  PRT
<213>  artificial

<220>
<223>  peptide

<400>  58

Gln Val Gln Leu Gln Glu Ser Gly Pro Gly Leu Val Lys Pro Gly Ala
1               5                   10                  15

Thr Val Lys Ile Ser Cys Lys Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  59
<211>  109
<212>  PRT
<213>  artificial

<220>
<223>  peptide

<400>  59

Gln Val Gln Leu Gln Glu Ser Gly Pro Gly Leu Val Lys Pro Gly Ala
1               5                   10                  15

Thr Val Lys Ile Ser Cys Lys Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  60
<211>  109
<212>  PRT
<213>  artificial

<220>
<223>  peptide

<400>  60

Glu Val Gln Leu Val Gln Ser Gly Ala Glu Val Lys Lys Pro Gly Glu
1               5                   10                  15

Ser Leu Arg Ile Ser Cys Lys Gly Ser Gly Tyr Thr Phe Thr Asn Tyr
            20                  25                  30

Tyr Val Phe Trp Ile Arg Gln Ser Pro Ser Arg Gly Leu Glu Trp Leu
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  61
<211>  109
<212>  PRT
<213>  artificial

<220>
<223>  peptide

<400>  61

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gly Tyr Thr Met Asp Tyr Trp Gly Gln Gly
            100                 105

<210>  62
<211>  109
<212>  PRT
<213>  artificial

<220>
<223>  peptide

<400>  62

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys
                85                  90                  95

Ala Arg Gly Gly Tyr Thr Met Asp Tyr Trp Gly Gln Gly
            100                 105

<210>  63
<211>  109
<212>  PRT
<213>  artificial

<220>
<223>  peptide

<400>  63

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Gly Gly Tyr Thr Met Asp Tyr Trp Gly Gln Gly
            100                 105

<210>  64
<211>  109
<212>  PRT
<213>  artificial

<220>
<223>  peptide

<400>  64

Glu Val Gln Leu Val Gln Ser Gly Ala Glu Val Lys Lys Pro Gly Ala
1               5                   10                  15

Thr Val Lys Ile Ser Cys Lys Val Ser Gly Tyr Thr Phe Thr Asn Tyr
            20                  25                  30

Tyr Val Phe Trp Ile Arg Gln Pro Pro Gly Lys Gly Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  65
<211>  109
<212>  PRT
<213>  artificial

<220>
<223>  peptide

<400>  65

Glu Val Gln Leu Val Gln Ser Gly Ala Glu Val Lys Lys Pro Gly Glu
1               5                   10                  15

Ser Leu Arg Ile Ser Cys Lys Gly Ser Gly Tyr Thr Phe Thr Asn Tyr
            20                  25                  30

Tyr Val Phe Trp Ile Arg Gln Ser Pro Ser Arg Gly Leu Glu Trp Leu

35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  66
<211>  109
<212>  PRT
<213>  artificial

<220>
<223>  peptide

<400>  66

Glu Val Gln Leu Val Gln Ser Gly Ala Glu Val Lys Lys Pro Gly Glu
1               5                   10                  15

Ser Leu Arg Ile Ser Cys Lys Gly Ser Gly Tyr Thr Phe Thr Asn Tyr
            20                  25                  30

Tyr Val Phe Trp Ile Arg Gln Ser Pro Ser Arg Gly Leu Glu Trp Leu
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  67
<211>  109
<212>  PRT
<213>  artificial

<220>

<223>  peptide

<400>  67

Gln Ile Thr Leu Lys Glu Ser Gly Pro Thr Leu Val Lys Pro Thr Gln
1               5                   10                  15

Thr Leu Thr Leu Thr Cys Thr Phe Ser Gly Tyr Thr Phe Thr Asn Tyr
            20                  25                  30

Tyr Val Phe Trp Val Arg Gln Ala Pro Gly Gln Gly Leu Glu Trp Met
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  68
<211>  109
<212>  PRT
<213>  artificial

<220>
<223>  peptide

<400>  68

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys

85                  90                  95

Ala Arg Gly Gly Tyr Thr Met Asp Tyr Trp Gly Gln Gly
            100                 105

<210>  69
<211>  109
<212>  PRT
<213>  artificial

<220>
<223>  peptide

<400>  69

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys
                85                  90                  95

Ala Arg Gly Gly Tyr Thr Met Asp Tyr Trp Gly Gln Gly
            100                 105

<210>  70
<211>  109
<212>  PRT
<213>  artificial

<220>
<223>  peptide

<400>  70

Gln Val Gln Leu Gln Glu Ser Gly Pro Gly Leu Val Lys Pro Ser Gln
1               5                   10                  15

Thr Leu Ser Leu Thr Cys Thr Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Leu Thr Ile Ser Lys Asp Thr Ser Lys Asn Gln Val Val
65                  70                  75                  80

Leu Thr Met Thr Asn Met Asp Pro Val Asp Thr Ala Thr Tyr Tyr Cys
                85                  90                  95

Ala Arg Gly Gly Tyr Thr Met Asp Tyr Trp Gly Gln Gly
            100                 105

<210>  71
<211>  109
<212>  PRT
<213>  artificial

<220>
<223>  peptide

<400>  71

Gln Val Gln Leu Gln Glu Ser Gly Pro Gly Leu Val Lys Pro Gly Ala
1               5                   10                  15

Thr Val Lys Ile Ser Cys Lys Val Ser Gly Tyr Thr Phe Thr Asn Tyr
            20                  25                  30

Tyr Val Phe Trp Val Arg Gln Ala Arg Gly Gln Arg Leu Glu Trp Ile
        35                  40                  45

Gly Asp Ile Asn Pro Val Asn Gly Asp Thr Asn Phe Asn Glu Lys Phe
    50                  55                  60

Lys Asn Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Thr Met Asp Tyr Trp Gly Gln Gly
            100                 105

<210>  72
<211>  337
<212>  PRT
<213>  artificial

<220>
<223>  peptide

<400>  72

Thr Leu Val Thr Val Ser Ser Ala Ser Thr Lys Gly Pro Ser Val Phe
1               5                   10                  15

Pro Leu Ala Pro Ser Ser Lys Ser Thr Ser Gly Gly Thr Ala Ala Leu
            20                  25                  30

Gly Cys Leu Val Lys Asp Tyr Phe Pro Glu Pro Val Thr Val Ser Trp
        35                  40                  45

Asn Ser Gly Ala Leu Thr Ser Gly Val His Thr Phe Pro Ala Val Leu
    50                  55                  60

Gln Ser Ser Gly Leu Tyr Ser Leu Ser Ser Val Val Thr Val Pro Ser
65                  70                  75                  80

Ser Ser Leu Gly Thr Gln Thr Tyr Ile Cys Asn Val Asn His Lys Pro
                85                  90                  95

Ser Asn Thr Lys Val Asp Lys Lys Val Glu Pro Lys Ser Cys Asp Lys
            100                 105                 110

Thr His Thr Cys Pro Pro Cys Pro Ala Pro Glu Leu Leu Gly Gly Pro
        115                 120                 125

Ser Val Phe Leu Phe Pro Pro Lys Pro Lys Asp Thr Leu Met Ile Ser
    130                 135                 140

Arg Thr Pro Glu Val Thr Cys Val Val Val Asp Val Ser His Glu Asp
145                 150                 155                 160

Pro Glu Val Lys Phe Asn Trp Tyr Val Asp Gly Val Glu Val His Asn
                165                 170                 175

Ala Lys Thr Lys Pro Arg Glu Glu Gln Tyr Asn Ser Thr Tyr Arg Val
            180                 185                 190

Val Ser Val Leu Thr Val Leu His Gln Asp Trp Leu Asn Gly Lys Glu
        195                 200                 205

Tyr Lys Cys Lys Val Ser Asn Lys Ala Leu Pro Ala Pro Ile Glu Lys
    210                 215                 220

Thr Ile Ser Lys Ala Lys Gly Gln Pro Arg Glu Pro Gln Val Tyr Thr
225                 230                 235                 240

Leu Pro Pro Ser Arg Asp Glu Leu Thr Lys Asn Gln Val Ser Leu Thr
                245                 250                 255

Cys Leu Val Lys Gly Phe Tyr Pro Ser Asp Ile Ala Val Glu Trp Glu
            260                 265                 270

Ser Asn Gly Gln Pro Glu Asn Asn Tyr Lys Thr Thr Pro Pro Val Leu
        275                 280                 285

Asp Ser Asp Gly Ser Phe Phe Leu Tyr Ser Lys Leu Thr Val Asp Lys
    290                 295                 300

Ser Arg Trp Gln Gln Gly Asn Val Phe Ser Cys Ser Val Met His Glu
305                 310                 315                 320

Ala Leu His Asn His Tyr Thr Gln Lys Ser Leu Ser Leu Ser Pro Gly
                325                 330                 335

Lys

<210>  73
<211>  245
<212>  PRT
<213>  artificial

<220>
<223>  peptide

<400>  73

Thr Leu Val Thr Val Ser Ser Ala Ser Thr Lys Gly Pro Ser Val Phe
1               5                   10                  15

Pro Leu Ala Pro Ser Ser Lys Ser Thr Ser Gly Gly Thr Ala Ala Leu
            20                  25                  30

Gly Cys Leu Val Lys Asp Tyr Phe Pro Glu Pro Val Thr Val Ser Trp
        35                  40                  45

Asn Ser Gly Ala Leu Thr Ser Gly Val His Thr Phe Pro Ala Val Leu
    50                  55                  60

Gln Ser Ser Gly Leu Tyr Ser Leu Ser Ser Val Val Thr Val Pro Ser
65                  70                  75                  80

Ser Ser Leu Gly Thr Gln Thr Tyr Ile Cys Asn Val Asn His Lys Pro
                85                  90                  95

Ser Asn Thr Lys Val Asp Lys Lys Val Glu Pro Lys Ser Cys Asp Lys
            100                 105                 110

Thr His Thr Cys Pro Pro Cys Pro Ala Pro Glu Leu Leu Gly Gly Pro
        115                 120                 125

Ser Val Phe Leu Phe Pro Pro Lys Pro Lys Asp Thr Leu Met Ile Ser
    130                 135                 140

Arg Thr Pro Glu Val Thr Cys Val Val Val Asp Val Ser His Glu Asp
145                 150                 155                 160

Pro Glu Val Lys Phe Asn Trp Tyr Val Asp Gly Val Glu Val His Asn
                165                 170                 175

Ala Lys Thr Lys Pro Arg Glu Glu Gln Tyr Gln Ser Thr Tyr Arg Val
            180                 185                 190

Val Ser Val Leu Thr Val Leu His Gln Asp Trp Leu Asn Gly Lys Glu
        195                 200                 205

Tyr Lys Cys Lys Val Ser Asn Lys Ala Leu Pro Ala Pro Ile Glu Lys
    210                 215                 220

Thr Ile Ser Lys Ala Lys Gly Gln Pro Arg Glu Pro Gln Val Tyr Thr
225                 230                 235                 240

Leu Pro Pro Ser Arg
                245

<210>  74
<211>  337
<212>  PRT
<213>  artificial

<220>
<223>  peptide

<400>  74

Trp Gly Gln Gly Thr Leu Val Thr Val Ser Ser Ala Ser Thr Lys Gly
1               5                   10                  15

Pro Ser Val Phe Pro Leu Ala Pro Cys Ser Arg Ser Thr Ser Glu Ser
            20                  25                  30

Thr Ala Ala Leu Gly Cys Leu Val Lys Asp Tyr Phe Pro Glu Pro Val
        35                  40                  45

Thr Val Ser Trp Asn Ser Gly Ala Leu Thr Ser Gly Val His Thr Phe
    50                  55                  60

Pro Ala Val Leu Gln Ser Ser Gly Leu Tyr Ser Leu Ser Ser Val Val
65                  70                  75                  80

Thr Val Pro Ser Ser Asn Phe Gly Thr Gln Thr Tyr Thr Cys Asn Val
                85                  90                  95

Asp His Lys Pro Ser Asn Thr Lys Val Asp Lys Thr Val Glu Arg Lys
            100                 105                 110

Cys Cys Val Glu Cys Pro Pro Cys Pro Ala Pro Pro Val Ala Gly Pro
        115                 120                 125

Ser Val Phe Leu Phe Pro Pro Lys Pro Lys Asp Thr Leu Met Ile Ser
    130                 135                 140

Arg Thr Pro Glu Val Thr Cys Val Val Val Asp Val Ser His Glu Asp
145                 150                 155                 160

Pro Glu Val Gln Phe Asn Trp Tyr Val Asp Gly Val Glu Val His Asn
                165                 170                 175

Ala Lys Thr Lys Pro Arg Glu Glu Gln Phe Asn Ser Thr Phe Arg Val
            180                 185                 190

Val Ser Val Leu Thr Val Val His Gln Asp Trp Leu Asn Gly Lys Glu
        195                 200                 205

Tyr Lys Cys Lys Val Ser Asn Lys Gly Leu Pro Ala Pro Ile Glu Lys
    210                 215                 220

Thr Ile Ser Lys Thr Lys Gly Gln Pro Arg Glu Pro Gln Val Tyr Thr
225                 230                 235                 240

Leu Pro Pro Ser Arg Glu Glu Met Thr Lys Asn Gln Val Ser Leu Thr
                245                 250                 255

Cys Leu Val Lys Gly Phe Tyr Pro Ser Asp Ile Ala Val Glu Trp Glu
            260                 265                 270

Ser Asn Gly Gln Pro Glu Asn Asn Tyr Asn Thr Thr Pro Pro Met Leu
        275                 280                 285

Asp Ser Asp Gly Ser Phe Phe Leu Tyr Ser Lys Leu Thr Val Asp Lys
    290                 295                 300

Ser Arg Trp Gln Gln Gly Asn Val Phe Ser Cys Ser Val Met His Glu
305                 310                 315                 320

Ala Leu His Asn His Tyr Thr Gln Lys Ser Leu Ser Leu Ser Pro Gly
                325                 330                 335

Lys

<210>  75
<211>  334
<212>  PRT
<213>  artificial

<220>
<223>  peptide

<400>  75

Thr Thr Val Thr Val Ser Ser Ala Ser Thr Lys Gly Pro Ser Val Phe
1               5                   10                  15

Pro Leu Ala Pro Cys Ser Arg Ser Thr Ser Glu Ser Thr Ala Ala Leu
            20                  25                  30

Gly Cys Leu Val Lys Asp Tyr Phe Pro Glu Pro Val Thr Val Ser Trp
        35                  40                  45

Asn Ser Gly Ala Leu Thr Ser Gly Val His Thr Phe Pro Ala Val Leu
    50                  55                  60

Gln Ser Ser Gly Leu Tyr Ser Leu Ser Ser Val Val Thr Val Pro Ser
65                  70                  75                  80

Ser Ser Leu Gly Thr Lys Thr Tyr Thr Cys Asn Val Asp His Lys Pro
                85                  90                  95

Ser Asn Thr Lys Val Asp Lys Arg Val Glu Ser Lys Tyr Gly Pro Pro
            100                 105                 110

Cys Pro Pro Cys Pro Ala Pro Glu Phe Leu Gly Gly Pro Ser Val Phe
        115                 120                 125

Leu Phe Pro Pro Lys Pro Lys Asp Thr Leu Met Ile Ser Arg Thr Pro
    130                 135                 140

Glu Val Thr Cys Val Val Val Asp Val Ser Gln Glu Asp Pro Glu Val
145                 150                 155                 160

Gln Phe Asn Trp Tyr Val Asp Gly Val Glu Val His Asn Ala Lys Thr
                165                 170                 175

Lys Pro Arg Glu Glu Gln Phe Asn Ser Thr Tyr Arg Val Val Ser Val
            180                 185                 190

Leu Thr Val Leu His Gln Asp Trp Leu Asn Gly Lys Glu Tyr Lys Cys
        195                 200                 205

Lys Val Ser Asn Lys Gly Leu Pro Ser Ser Ile Glu Lys Thr Ile Ser
    210                 215                 220

Lys Ala Lys Gly Gln Pro Arg Glu Pro Gln Val Tyr Thr Leu Pro Pro
225                 230                 235                 240

Ser Gln Glu Glu Met Thr Lys Asn Gln Val Ser Leu Thr Cys Leu Val
                245                 250                 255

Lys Gly Phe Tyr Pro Ser Asp Ile Ala Val Glu Trp Glu Ser Asn Gly
            260                 265                 270

Gln Pro Glu Asn Asn Tyr Lys Thr Thr Pro Pro Val Leu Asp Ser Asp
        275                 280                 285

Gly Ser Phe Phe Leu Tyr Ser Arg Leu Thr Val Asp Lys Ser Arg Trp
    290                 295                 300

Gln Glu Gly Asn Val Phe Ser Cys Ser Val Met His Glu Ala Leu His
305                 310                 315                 320

Asn His Tyr Thr Gln Lys Ser Leu Ser Leu Ser Leu Gly Lys
                325                 330

<210>  76
<211>  334
<212>  PRT
<213>  artificial

<220>
<223>  peptide

<400>  76

Thr Thr Val Thr Val Ser Ser Ala Ser Thr Lys Gly Pro Ser Val Phe
1               5                   10                  15

Pro Leu Ala Pro Cys Ser Arg Ser Thr Ser Glu Ser Thr Ala Ala Leu
            20                  25                  30

Gly Cys Leu Val Lys Asp Tyr Phe Pro Glu Pro Val Thr Val Ser Trp

35                  40                  45

Asn Ser Gly Ala Leu Thr Ser Gly Val His Thr Phe Pro Ala Val Leu
    50                  55                  60

Gln Ser Ser Gly Leu Tyr Ser Leu Ser Ser Val Val Thr Val Pro Ser
65                  70                  75                  80

Ser Ser Leu Gly Thr Lys Thr Tyr Thr Cys Asn Val Asp His Lys Pro
                85                  90                  95

Ser Asn Thr Lys Val Asp Lys Arg Val Glu Ser Lys Tyr Gly Pro Pro
            100                 105                 110

Cys Pro Pro Cys Pro Ala Pro Glu Phe Glu Gly Gly Pro Ser Val Phe
        115                 120                 125

Leu Phe Pro Pro Lys Pro Lys Asp Thr Leu Met Ile Ser Arg Thr Pro
    130                 135                 140

Glu Val Thr Cys Val Val Val Asp Val Ser Gln Glu Asp Pro Glu Val
145                 150                 155                 160

Gln Phe Asn Trp Tyr Val Asp Gly Val Glu Val His Asn Ala Lys Thr
                165                 170                 175

Lys Pro Arg Glu Glu Gln Phe Asn Ser Thr Tyr Arg Val Val Ser Val
            180                 185                 190

Leu Thr Val Leu His Gln Asp Trp Leu Asn Gly Lys Glu Tyr Lys Cys
        195                 200                 205

Lys Val Ser Asn Lys Gly Leu Pro Ser Ser Ile Glu Lys Thr Ile Ser
    210                 215                 220

Lys Ala Lys Gly Gln Pro Arg Glu Pro Gln Val Tyr Thr Leu Pro Pro
225                 230                 235                 240

Ser Gln Glu Glu Met Thr Lys Asn Gln Val Ser Leu Thr Cys Leu Val
                245                 250                 255

Lys Gly Phe Tyr Pro Ser Asp Ile Ala Val Glu Trp Glu Ser Asn Gly
            260                 265                 270

Gln Pro Glu Asn Asn Tyr Lys Thr Thr Pro Pro Val Leu Asp Ser Asp
        275                 280                 285

Gly Ser Phe Phe Leu Tyr Ser Arg Leu Thr Val Asp Lys Ser Arg Trp
    290                 295                 300

Gln Glu Gly Asn Val Phe Ser Cys Ser Val Met His Glu Ala Leu His
305                 310                 315                 320

Asn His Tyr Thr Gln Lys Ser Leu Ser Leu Ser Leu Gly Lys
                325                 330

<210>  77
<211>  16
<212>  PRT
<213>  artificial

<220>
<223>  peptide

<400>  77

Arg Ser Ser Gln Ser Leu Val His Ser Asn Gly Asn Thr Tyr Leu His
1               5                   10                  15

<210>  78
<211>  7
<212>  PRT
<213>  artificial

<220>
<223>  peptide

<400>  78

Lys Val Ser Tyr Arg Phe Ser
1               5

<210>  79
<211>  9
<212>  PRT
<213>  artificial

<220>
<223>  peptide

<400>  79

Ser Gln Asn Thr His Val Pro Arg Thr
1               5

<210>  80
<211>  10
<212>  PRT
<213>  artificial

<220>
<223>  peptide

<400>  80

Gly Tyr Thr Phe Thr Asn Tyr Tyr Val Phe
1               5                   10

<210>  81
<211>  16
<212>  PRT
<213>  artificial

<220>
<223>  peptide

<400>  81

Asp Ile Asn Pro Val Asn Gly Asp Thr Asn Phe Asn Glu Lys Phe Lys
1               5                   10                  15

<210>  82
<211>  7
<212>  PRT
<213>  artificial

<220>
<223>  peptide

<400>  82

Gly Gly Tyr Thr Met Asp Tyr
1               5

## Description

The present invention relates to CD47 antibodies or fragments thereof binding specifically to the CD47 immunogen for use in preventing or treating tissue injury in states of ischemia or reperfusion in an object whereby the CD47 antibodies or fragments thereof are introduced intravascularly at the or near by the place of ischemia or reperfusion.

### Prior Art

CD47, first detected on ovarian carcinoma and also known as integrin-associated protein (TAP) is widely expressed in many cell types including blood cells, neurons and cardiomyocytes. The levels of CD47 are significantly increased in many human cancers including solid tumors and hematopoetic diseases. Actually, high expression patterns have been associated with poor prognosis of the subjects. The correlation of CD47 levels and disease inspired to develop target therapies using humanized antibodies directed against tumor cells expressing increased CD47.

The CD47 molecule is a member of the immunoglobulin superfamily and its structure is unique insofar that it contains five transmembrane regions, a single Ig-like extracellular domain and a short intracellular tail.

However, regulation mechanism of the CD47 expression is still under investigation. Of note, CD47 expression has been related to states of hypoxia. In experimental renal ischemia or pulmonary artery hypertension CD47 levels were significantly elevated (Soto-Pantoja, D.R., et al., Expert Opinions on Therapeutic Targets, 2013, 17:89-103). However, the role of CD47 in the heart remains incompletely determined.

Two major functions of CD47 are presently discussed. The first major function of CD47 is the functional receptor for thrombospondines (TSP). The TSP family consists of five multimeric, multidomain calcium-binding extracellular matrix proteins. Upon binding to CD47, TSP regulates several processes within the cardiomyocyte including calcium homoeostasis, gene expression programs, the regulation of matrix metalloproteins and NO signaling. Emerging evidence appoints to a significant role for TSP in cardiac disease and increased levels have been found in pressure-load hypertrophie, heart failure and myocardial infarction. It is well known that NO has beneficial effects in myocardial functions in a dose dependent manner.

The second major function of CD47 is the regulation of macrophages. Interaction of CD47 with signal regulatory protein (SIRPα) on macrophages conveys a signaling and a recognition of cells meaning that the cell signals to the macrophages that they are self and should not be phagocyzed. Cells with low CD47 surface levels as in aged erythrocytes or with antibody-blocked CD47 receptor are primed for removal by the phagocytic machinery while tumor cells with high CD47 pattern avoid clearance. SIRPα represents an inhibitory receptor as a counterpart of CD47 expressed on phagocytes. Upon binding of CD47 by SIRPα a phosphorylation cascade is triggered downregulating the activity of the macrophages. In acute myocardic infarction (AMI) the recruitment of adequate phagocytosis is required. That is, NO-dependent manipulation of macrophage inhibitory factor (MIF), a component involved in macrophage attraction in diseased organs, could contribute to infarct reduction. On the other hand, macrophage malfunction can be deleterious. Phagocytes including macrophages interacting with the damaged cardiomyocytes resulting in proteolysis, dead-cell removal, angiogenesis and wound healing allowing effecting of remodeling. Actually a timely removal of apotopic cells in the border zones of the AAR (area at risk) thus preventing the dissemination of injury throughout the myocardium. Based on tumor studies, CD47 has emerged a major regulator of macrophage function.

In EP 2 569 013 B1 humanized and chimeric monodonal antibodies against CD47 are described being beneficial in the treatment of cancer like acute myeloid leukemia. Further, EP 2 477 648 A1 describes a synergistic anti-CD47 therapy for hematologic cancers.

In WO 2015/191861 A1 therapeutic CD47 antibodies have been described blocking the binding of SIRPα and TSP1 to cancer, thus, promoting phagocytosis of susceptible cancer cells. Therein, also the use of the therapeutic CD47 antibodies in other diseases including treatment of ischemia/reperfusion injury, autoimmune or inflammatory disease, as well as various types of cancer have been identified without demonstrating any effect on the same except for cancer.

Reperfusion injury also known as re-oxygenation injury is the tissue damage caused when blood supply returns to the tissue after a period of ischemia or lack of oxygen, namely anoxia or hyperoxia. That is, absence of oxygen and nutrients from blood during the ischemic period may damage said tissue, thus, restoration of circulation results in inflammation and oxidative damage through the induction of oxidative stress rather than restoration of malfunction. Various types of reperfusion injury exist including various mechanisms. The treatment of reperfusion injury includes various approaches including therapeutic hypothermia and treatment with various compounds including cyclosporine.

Ischemia is known as a restriction in the blood supply to tissues, thus, causing a shortage of oxygen as well as nutrients required for cellular metabolism. Ischemia typically results in damage or disfunction of the surrounding tissue. Ischemia may occur in various of the bodies including cardiac ischemia also known as myocardial infarction as wells as in brain (stroke) in the bowel or the limb.

Although various approaches for treating ischemia and reperfusion are described in the art, there is still ongoing need to treat ischemia and reperfusion, thus, reducing damaging of tissue. In particular, early treatment is desired.

### Brief description of the present invention

In a first aspect, the present invention relates to CD47 antibodies or fragments thereof binding specifically to the CD47 immunogen for use in preventing or treating tissue injury in states of ischemia and reperfusion in an object whereby the CD47 antibodies or fragments thereof are introduced intravascularly at the or near by the place of ischemia or reperfusion.

It has been recognized that administering the CD47 antibodies or fragments thereof intravascularly at or near by the place of ischemia or reperfusion increase the efficacy of the inhibition of tissue damage or tissue injury. Thus, the acute injury phase will be limited.

In a further aspect, the present invention relates to a method for preventing or treating tissue injury in states of ischemia and reperfusion including the step of administering or introducing intravascularly at the or near by the place of ischemia or reperfusion CD47 antibodies or fragments thereof biding specifically to the CD47 immunogene.

### Brief description of the drawings

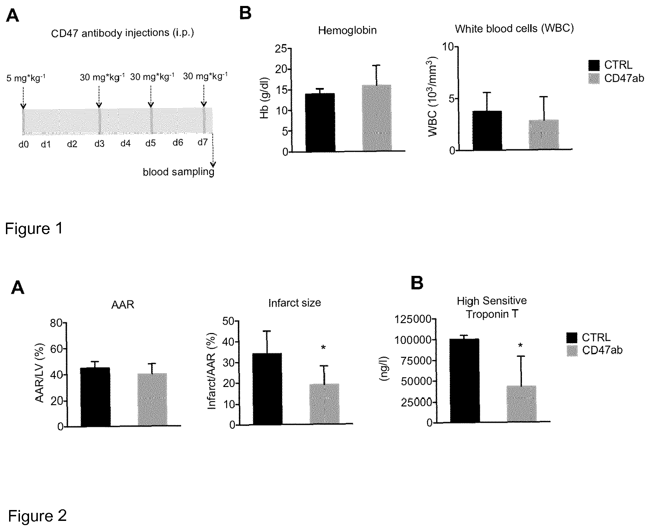

Figure 1.: Effect of a CD47 therapeutic regimen on blood cell counts. (A) Experimental scheme. Mice were treated by i.p. injection with CD47 antibody at the shown time intervals. Blood was taken 90 min after the last injection. (B) No differences were detectable for hemoglobin and white blood cells in CD47Ab treated animals vs. control littermates (n=5, p>0.05, unpaired student's t-test, graph shows mean ± s.d.).
Figure 2.: Anti-CD47 antibody administration reduces infarct sizes. (A) Following anti-CD47Ab treatment or control IgG, infarct per AAR was significantly reduced vs. CTRL animals, while AAR was comparable in both groups. (B) This was mirrored by significantly reduced troponin levels as measured with a high sensitive assay as marker for myocardial injury. (*p<0.05, unpaired student's t-test, n=7 vs. 6, data are mean ± s.d.)

### Detailed description of the present invention

In the first aspect, the present invention relates to CD47 antibodies or fragments thereof binding specifically to the CD47 immunogen for use in preventing or treating tissue injury in states of ischemia and reperfusion in an object whereby the CD47 antibodies or fragments thereof are introduced intravascularly at the or near by the place of ischemia or reperfusion.

The present inventors recognized that tissue damage following ischemia/reperfusion can be reduced or inhibited by targeting CD47 in the vicinity of the area of ischemia or reperfusion. By binding to the cell surface receptor CD47 enhancing CD47 SERPα signaling the tissue injury in states of ischema and reperfusion can be reduced. In particular, it is possible to prophylactically or therapeutically treat tissue injury as a result of various disorders, conditions or diseases. Importantly, the present inventors recognized that only when introducing the CD47 antibodies or fragments thereof binding specifically to the CD47 immunogen intravascularly at or near by the place of ischemia or reperfusion, reduction of tissue injury can be achieved. It has been recognized by the inventors that the reduction of tissue injury relies on the activation of endogenous phagocytic machinery for the removal of apoptotic cells in the promotion of adequate wound healing.

As used herein, the term "treatment" or "treating" refer to both the therapeutic treatment and prophylactic or preventative measures unless otherwise identified. Those in need of treatment include those already with the disorder as well as those in which the disorder is to be prevented or reducing the tissue injury and inhibiting any tissue injury.

The term "antibody" is used in the process and specifically covers monoclonal antibodies (including full length monoclonal antibodies), polyclonal antibodies and multi-specific antibodies (e.g. bi-specific antibodies) and antibody fragments so long as they inhibit the desired biological activity.

As used in this invention, the term "epitope" means any antigenic epitope determining an antigen to which the epitope of an antibody binds. Epitope determines usually consists of chemical active surface, groupings of molecules such as amino acids or sugar side chains and usually have specific three-dimensional structural characteristics, as well as specific charged characteristics.

The term "antibody fragment" and all variants thereof as used herein are defined as a portion of an intact antibody comprising the antigen binding side or variable region of the intact antibody. For example, the fragment is free of that constant heavy chain domains of the region of the intact antibody. Examples of any antibody fragments are Fab, Fab', Fab'-SH, F(ab')₂ and FV fragments; diabodies as well as single chain antibody fragments or single chain polypeptides.

The term "monoclonal antibody" as used herein refers to an antibody obtained from a population of substantially homogeneous antibody, i.e. the individual antibodies comprising the population of identical except for possible naturally occurring limitations that may be present in minor amounts. Monoclonal antibodies are highly specific, being directed against a single antigenic site. Each monoclonal antibody is directed against a single determinant on the antigen. In addition to their specificity, the monoclonal antibodies are advantageous in that they can be synthetized by hybridoma culture without any contamination with immunoglobulins. The monoclonal antibodies herein include hybrid and recombinant antibodies produced by splicing of a variable domain of anti-CD47 antibody with a constant domain, e.g. humanized antibodies, or a light-chain with a heavy chain, or a chain from one species with a chain form another species, or fusion with heterologous proteins, regard as of species of origin or immunoglobulin class or subclass designation as well as antibody fragments, as long as they exhibit the desired biological inhibit.

"Reperfusion" refers to restoration of blood flow to tissue that is ischemic, due to decreased blood flow. Reperfusion is a procedure for treating infarction or other ischemia, by enabling viable ischemic tissue to recover, thus limiting further necrosis. However, as noted before, reperfusion can itself further damage the ischemic tissue, causing the reperfusion injury. Further, ischemic/ reperfusion injury involves tissue injury that occurs after blood flow is restored.

"Ischemia" refers to a vascular phenomenon in which a decrease in the blood supply to a bodily organ, tissue or part is caused, for instance by constriction or obstruction of one or more blood vessels. Ischemia sometimes results from vasoconstriction or thrombosis or embolism. Ischemia can lead to direct ischemic injury, tissue damage due to cell that is caused by reduces oxygen supply. Ischemia can occur acutely, as during surgery or from trauma to tissue incurred in accidents, injuries and wall settings, or following harvest of organ intended for subsequent transportation. It can also occur sub-acutely, as found in arteriosclerotic peripheral vascular disease, where progressive constriction of blood vessels leads to inadequate blood flow to tissue and organs. The resulted injury after transit decrease or interruption of blood flow involves two components, the direct injury occurring to ischemic interval and the indirect or reperfusion injury that follows.

The phrase "humanized antibodies" refers to monoclonal antibodies and antigen binding fragments thereof, including the antibody compounds described herein, that have binding in functional properties according to the disclosure similar to those disclosed herein and that have framework in consent regions that are substantially human or fully human surrounding CDRs deriving from a non-human antibody. In this connection, the term "framework region" or "framework sequence" refers to any one of the framework regions 1 to 4. Humanized antibodies and antigen binding fragments encompassed by the present disclosure include molecules wherein any one or more of the fragment regions 1 to 4 is substantially or fully human, i.e. wherein any of the possible combinations of individuals substantially or fully framework regions 1 to 4 is present. Substantially human frameworks are those that have at least 80% sequence identity to a known human germline framework sequence, preferably that substantially human framework have at leat 85%, at least 88%, at least 90%, at least 91%, at least 92%, at least 93%, at least 94%, at least 95%, at leaste 96%, at least 97%, at least 98% or at least 99% sequence identity to a framework sequence as described or to a known human germline framework sequence.

As used herein, the phrase "sequence identity" means a percentage of identical nucleotide or amino acid residues at corresponding positions in two or more sequences when the sequence are known to maximize sequence matching, i.e. taking into account gaps and insertions. The skilled person is well aware of suitable methods and programs allowing calculation thereof accordingly, the same are publically available.

The terms "specifically combines", "binds specifically", "specific binding" and the like as applied to the present antibody compounds refer to the ability of a specific binding agent, the antibody or fragments thereof, to bind to a target molecular species containing the epitope in preference to binding to other molecular species with which the specific binding agent and target molecular species are mixed. A specific binding agent is said specifically to recognize a target molecular species when it can bind specifically to that target containing the epitope.

The singular terms "a", "an" and "the" include plural reference under this context clearly indicates otherwise.

The term "comprising" as used herein is open-ended and means that the subject matter must contain all the features specifically recited therein, but there is no bar or additional features that are not recited being present as well. The term "comprising" or "comprise" are used interchangeably herein with the term "contain". A specific embodiment of comprising or containing is consisting of.

The phrase "introduced... at the or near by the place of ischemia or reperfusion" refers to an administration or introduction of the antibodies or fragments thereof into the place of ischemia or reperfusion or in the vicinity of said place, thus, the antibodies or fragments thereof administered come immediately into contact with the place of ischemia or reperfusion. Thus, any deterioration or degradation of the effective agents, namely, the antibodies or fragments thereof, is avoided. The present inventors recognized that immediate administration is beneficial to reduce any tissue injury in states of ischemia reperfusion accordingly.

In an embodiment of the present invention, the object is a human and the CD47 immunogen is the immunogen having the sequence of SEQ ID No. 2 encoded e.g. by a nucleic acid sequence of SEQ ID No. 1 and CD47 antibodies or fragments thereof are humanized antibodies or fragments thereof.

It is preferred that the antibodies or the fragments thereof binding specifically to the CD47 immunogen of the object are modified in a way that the antibodies are tolerable by the object to be treated.

In an embodiment the object is a mammal, in particular a human.

In a further aspect, the present invention relates to the use of the CD47 antibodies or fragments thereof preventing or treating tissue injury in states of ischemia or reperfusion whereby the introduction or administration of said CD47 antibodies or fragments thereof into the object is by catheter, by infusion and/or other intravenous administration, by intraventricular administrations or combinations thereof.

For example, the introduction is by catheter into the reperfused coronary artery. Alternatively, introduction or administration is by both introduction of a loading dose by catheter into the coronary artery followed by a continuous intravenous infusion for a predetermined time, e.g. for 1 hour, for 12 hours, for 24 hours, for 2 days or 3 days. Alternatively, the administration is by intravenous injection of a bolus followed by continues infusion. In another embodiment, the introduction is by intraventricular injection of a bolus or by intraventricular injection followed by a continuous intravenous infusion.

For example, the administration is regionally at the or near by the place of ischemia or reperfusion within the first 30 minutes, like the first 15 minutes, after ischemia or reperfusion and/or is followed by a continuous infusion.

In a further embodiment, the present invention relates to the CD47 antibodies or fragments thereof for use in preventing or treating tissue injury in states of ischemia or reperfusion wherein the ischemia and reperfusion is a result of
i) Myocardial infarction,
ii) Cardiac arrest and resuscitation,
iii) Coronary bypass surgery under the use of an extracorporal life support systems,
iv) Treatment of chronic coronary total occlusion lesions,
v) Treatment of type B aortic dissection using catheter interventional techniques,
vi) Stroke and interventional stroke treatment,
vii) Cardiogenic shock,
viii) Heart transplantation,
ix) Replacement of aortic valves or
x) High risk coronary interventions under the use of circulatory support systems.

In a further embodiment, the present invention relates to the CD47 antibodies or fragments thereof for use in preventing or treating tissue injury in states of ischemia or reperfusion in an object wherein the treatment is a prophylactic treatment before heart transplantation or replacement of vessels, in particular, artery and veins including aorta. As demonstrated in the examples, a prophylactic administration of the CD47 antibodies or fragments thereof induces significantly the area at risk in case of ischemia or reperfusion. Thus, any adverse effects due to the heart transplantation or replacement of vessels, in particular, artery and veins including aorta is reduced.

The skilled person is well aware of suitable methods for the prophylactic treatment, in particular, the prophylactic treatment before heart transplantation or replacement of vessels. Typically, the administration is at the or nearby the place of transplantation or replacement of vessels and, in addition, the transplanted organ, like the heart, or the newly vessel, including artery and veins, are prophylactically treated with the CD47 antibodies or fragments thereof binding specifically to the CD47 immunogen.

In a further embodiment, the present invention relates to the CD47 antibodies or fragments thereof for use in preventing or treating tissue injury in states of ischemia or reperfusion in an object undergoing treatment, in particular, surgery, including extracorporal support systems. That is, to avoid or diminish any adverse side effects during treatment, the prophylactic or therapeutic administration of the CD47 antibodies or fragments thereof according to the present invention are beneficial. As identified, it is necessary to administrate the same at the or nearby the place of possible ischemia or reperfusion.

The term "at the or nearby the place of ischemia or reperfusion" includes embodiments, in particular, when administrated prophylactically, where the possible place of ischemia or reperfusion is considered to take place. Said term include embodiments where the administration is within or nearby the AAR.

In a further embodiment, the CD47 antibodies or fragments thereof according to the present invention are for use in preventing or treating tissue injury in states of ischemia or reperfusion wherein the intravascular introduction thereof at the or nearby the place of ischemia or reperfusion is in concert with an infusion of said CD47 antibodies or fragments.

That is, beside the local or regional initial treatment, the continued treatment is systematically, i.e. by infusion.

While the first, initial, prophylactic or therapeutic treatment is a local administration of the antibodies or fragments thereof, the further treatment is e.g. by continuous administration either systematically or locally. The skilled person is well aware of the suitable methods accordingly.

Further, the present invention provides the use of the CD47 antibodies or fragments thereof wherein the administration by intervascular introduction at the or nearby the place of ischemia or reperfusion is simultaneously or sequentially with the infusion of the CD47 antibodies or fragments thereof.

Moreover, the CD47 antibodies or fragments thereof for use in preventing or treating tissue injury in states of ischemia and reperfusion in an object is characterized in that the CD47 antibodies or fragments thereof are administrated by catheter at the or nearby the place of ischemia or reperfusion, in particular, a damaged organ, in a concentration sufficient to locally inhibit CD47 signaling, thus, enabling removing damaged cells by a pharmaceutic machinery for improving reperfusion injury.

That is, the present inventors recognized that after ischemia, CD47 expression is increased, thus, the "don't eat me" signal of the cells identifies themselves as cells not ready for phagocytosis by macrophages or the phagocytes.

In addition, the ischemia and or reperfusion increased at CD47 expression not in the area of the severest damages, the high levels, are present in the boarder zones of the tissue damage (of the AAR) whereby these boarder zones are particularly important for the effects of cardio protective measures/therapies and infarct-sparing effects are believed to be localized in this area.

Hence, the method and use according to the present invention targets the most important region, namely, the boarder zones of the damaged tissue or AAR, thus, inhibiting any spreading or propagation with the damaged tissue accordingly. Further, a wound healing is improved due to the activation of the phagocytic machinery or removing apoptotic cells and, in addition, promoting the adequate wound healing.

Also the increasing survive of issue make tissue by targeting CD47 has been described before, e.g. Isenberg, J.S. at al, circulation research, 2007; 100; 712-720, only the present inventors recognized that in a first step the CD47 antibodies or fragments thereof are introduced intravascularly at the or nearby the place of ischemia or reperfusion.

Hence, in an embodiment the CD47 antibodies or fragments thereof are for use in preventing or treating tissue injury in states of ischemia or reperfusion in an object wherein the administration or introduction is by a combination of a first administration of an initiate dosage in form of intraventricular injection of a bolus or intravenous injection of a bolus or by catheter to the coronary artery followed by continuous infusion.

Moreover, the CD47 antibodies or fragments thereof particularly useful according to the present invention are CD47 antibodies or fragments thereof as described e.g. in WO 2011/143624 A2, in WO 2011/034969 A1 as well as WO 2015/191861 A1.

That is, in an embodiment of the present invention, the CD47 antibodies or fragments thereof for use in preventing or treating tissue injury in states of ischemia or reperfusion in an object an isolated chimeric or humanized antibody that specifically bind to human CD47, wherein said antibody comprises a heavy chain having each of the CDR sequences set forth in SEQ ID Nos. 3, 4, 5, 6, 7, 8, 9, 10, 11 and a light chain having each of the CDR sequences set forth in SEQ ID No. 12, 13, 14, 15, 16, 17, 18, 19, 20.

The CD47 antibodies or fragments thereof for use in preventing or treating tissue injury in states of ischemia or reperfusion in an object according to any one of claims 1 to 11 wherein the CD47 antibodies or fragments thereof are a monoclonal antibody or antigen binding fragment thereof which comprises a light chain variable region (LCVR) and a heavy chain variable region (HCVR), wherein said LCVR and said HCVR comprise, respectively, amino acid sequ3ences selected from among the following combinations of LCVRs and HCVRs:
wherein each one of the LCVR SEQ ID Nos 21 to 45 further comprises a constant domain having the amino acid sequence shown in SEQ ID No 46, and
wherein each one of the HCVR SEQ ID Nos 47 to 71 comprises a constant domain selected from among SEQ ID Nos 72, 73, 74, 75, 76.

The CD47 antibodies or fragments thereof for use in preventing or treating tissue injury in states of ischemia or reperfusion in an object according to claim 13 wherein the antibody or antigen binding fragment thereof comprises three light chain complementary determining regions (LCDRs 1 - 3) and three heavy chain complementary determining regions (HCDRs 1 - 3), wherein:
LCDR 1 comprises the amino acid sequence RSSQSLVHSNGNTYLH (SEQ ID NO: 77)
LCDR 2 comprises the amino acid sequence KVSYRFS (SEQ ID NO:78); and
LCDR 3 comprises the amino acid sequence SQNTHVPRT (SEQ ID NO:79);
HCDR1 comprises the amino acid sequence GYTFTNYYVF (SEQ ID NO:80);
HCDR 2 comprises the amino acid sequence DINPVNGDTNFNEKFKN (SEQ ID NO:81); and
HCDR 3 comprises the amino acid sequence GGYTMDY (SEQ ID NO:82).

In addition, the present invention relates to a method for treating ischemia or reperfusion in an object in need thereof for preventing or treating the tissue injury in states of ischemia or reperfusion comprising the step of administrating the CD47 antibodies or fragments thereof as defined herein, in particular, administrating the same intravascularly at the or near by the place of ischemia or reperfusion. The CD47 antibodies or fragments thereof binding specifically to the CD47 immunogen are administrated in effective dosages, that is, dosages are effected in preventing or treating the tissue injury in states of ischemia or reperfusion accordingly.

The skilled person is well aware of suitable dosages and can easily determine the effective amount accordingly.

Typically, the antibodies or fragments thereof are administrated as a pharmaceutically composition. The pharmaceutically compositions can be prepared by known methods. Typically, the pharmaceutic composition comprises a pharmaceutically or veterinary acceptable, e.g. physiologically acceptable, carrier, diluent or excipient.

As noted, the administration is intravascularly at the or nearby the place of ischemia or reperfusion. Of course, a combination with other known therapeutic methods are encompassed herein including the use of other antibodies or other active ingredients for preventing or treating the tissue injury in states of ischemia or reperfusion in an object accordingly.

The present invention will be described further by way of examples without limiting the same.

### Example 1 Effect of CD47 antibody administration on hemoglobin and white blood cells

Following a seven day administration of CD47 antibody by i.v. injection blood samples had been acquired to determine the blood cell count of all blood cells in conjunction with hemoglobin levels, which had been compared to pretreatment levels. Additionally, blood cell counts and hemoglobin levels will had been assessed in every trial conducted under the usage of CD47 receptor blocking therapies.

Previous studies in various preclinical cancer models have demonstrated that antibodies against CD47 can eliminate cancer cells or inhibit progression effectively. Herein, CD47 antibody administration had little to no side effects. Arguably, one of the highly discussed caveats of this approach is the interaction of the antibody with the recipient's red blood cells. Long-term blockade of CD47 on erythrocytes could enhance the clearance of these cells leading to anemia. Although this has not been the case in a relevant manner in the current preclinical tumor and transplantation studies, it was first set out to evaluate the effects on blood cells in mice after a seven day treatment with CD47 antibodies. Figure 1A shows the therapeutic regimen schematically. C57BL/6 mice (twelve ± three weeks old, male gender) were treated with an intraperitoneal (i.p.) injection of anti-CD47 antibody (CD47 ab, MIAP 301, Santa Cruz) on day 0 with a priming dose (5 mg*kg-1) and on days 3,5 and 7 (30 mg*kg-1) with a full therapeutic dose. This was compared against control injections at similar intervals. No injection was administered on days 1,2, 4 and 6. 90 min after the final dose, full blood was taken from the mouse heart, mixed with EDTA for anticoagulation and analyzed by flow cytometry. Figure 1B shows the respective results after the 7d regimen for hemoglobin and white blood cells. The applicant could demonstrate that CD47Ab treatment did not affect these levels in comparison to CTRL littermates. This is a prerequisite for subsequent infarction studies.

As demonstrated, the CD47 treatment using the antibodies described did not affect the level of hemoglobin and white blood cells in control groups being a sign for usability of the same in the prevention or treatment of tissue injury in states of ischemia or reperfusion.

### Example 2:

Based on the findings in Example 1, the efficacy of CD47 blockade was next tested in a mouse model of regional myocardial infarction. Mice received anti-CD47Ab according to the scheme outlined in fig.1. 90 min after the last injection, anesthesia was initiated.15 A thoracotomy was performed in intubated and mechanically ventilated mice. I/R was induced by reversible ligation of the left coronary artery. Ischemia was maintained for a period of 30 min after which the suture was released. After surgical wound closure, mice recovered for 24 h. Hereafter, hearts were excised and treated according to previously published protocols (Rassaf et al. Circulation Res 2014 May 9; 114(10):1601-10. Doi: 10.1161/CIRCRESAHA.114303822).15 Infarct size was calculated against the AAR. Calculation was performed by two operators. Fig. 2A shows that anti-CD47Ab treatment reduced infarct size significantly with no significant difference on the size of the AAR as control. In parallel this caused a reduction in troponin levels as measured in the blood of the respective animals (Fig. 2B).

As noted before, hypoxia is believed to enhance CD47 expression. Surprisingly, also ischemia/reperfusion with a 24 h reoxygenation of the myocardium at risk induces CD47 expression in a relevant manner. The applicants therefore characterized CD47 levels in mouse hearts following in vivo I/R and 24 h of reperfusion. Western blotting was performed using mouse whole heart homogenates and anti-CD47 showing that CD47 was increased after I/R as compared to baseline control hearts. In contrast to what is expected, I/R increased CD47 expression not in the area of the severest hypoxia (inner myocardial borders), but at high levels at the border zones (data not shown). These border zones are particularly important for the effects of cardioprotective measures/therapies and infarct-sparing effects are believed to be localized at this area.

As demonstrated further, the efficacy of CD47 blocking agents harder can be determined in regional myocardial infarction. That is, as demonstrated with myocardial ischemia/reperfusion, a prophylactic treatment reduces significantly the infarct size compared to none retreated animals.

Taken together, the present inventors found that blocking of CD47 with a specific antibody therapy has no side effects on blood cell counts following the chosen application regimen. Furthermore antibody-dependent blockade of CD47 results in an acute decrease in myocardial I/R injury in an in vivo mouse regional model.

## Claims

1. CD47 antibodies or fragments thereof binding specifically to the CD47 immunogen for use in preventing or treating tissue injury in states of ischemia and reperfusion in an object whereby the CD47 antibodies or fragments thereof are introduced intravascularly at the or near by the place of ischemia or reperfusion.

2. The CD47 antibodies or fragments thereof for use in a method of preventing or treating tissue injury in states of ischemia or reperfusion according to claim 1 wherein the object is a human and the CD47 immunogen is the immunogen having the sequence of SEQ ID No. 2 e.g. encoded by a sequence of SEQ ID No. 1 and the CD47 antibodies or fragments thereof are humanized antibodies or fragments thereof.

3. The CD47 antibodies or fragments thereof for use in preventing or treating tissue injury in states of ischemia or reperfusion according to any one of the preceding claims wherein the introduction is by catheter, by infusion and/or other intravenous administration, by intraventricular administration or combinations thereof..

4. The CD47 antibodies or fragments thereof for use in preventing or treating tissue injury in states of ischemia or reperfusion according to any one of the preceding claims wherein the ischemia and reperfusion is a result of
xi) Myocardial infarction,
xii) Cardiac arrest and resuscitation,
xiii) Coronary bypass surgery under the use of an extracorporal life support systems,
xiv) Treatment of chronic coronary total occlusion lesions,
xv) Treatment of type B aortic dissection using catheter interventional techniques,
xvi) Stroke and interventional stroke treatment,
xvii) Cardiogenic shock,
xviii) Heart transplantation,
xix) Replacement of aortic valves or
xx) High risk coronary interventions under the use of circulatory support systems.

5. The CD47 antibodies or fragments thereof for use in preventing or treating tissue injury in states of ischemia or reperfusion according to any one of the preceding claims whereby administration is regionally at the or near by the place of ischemia or reperfusion within the first 30 minutes, like the first 15 minutes after ischemia or reperfusion and/or is followed by continuous infusion.

6. The CD47 antibodies or fragments thereof for use in preventing or treating tissue injury in states of ischemia or reperfusion in an object according to any one of the preceding claims wherein the treatment is a prophylactic treatment before heart transplantation or replacement of vessels, in particular, artery and veins including aorta.

7. The CD47 antibodies or fragments thereof for use in preventing or treating tissue injury in states of ischemia or reperfusion in an object according to any of the preceding claims undergoing treatment, in particular surgery, including extracorporal support systems.

8. The CD47 antibodies or fragments thereof for use in preventing or treating tissue injury in states of ischemia or reperfusion according to any one of the preceding claims wherein the intravascular introduction thereof at the or near by the place of ischemia or reperfusion is in concert with an infusion of the said CD47 antibodies or fragments thereof.

9. The CD47 antibodies or fragments thereof for use according to claim 8 wherein the administration by intravascular introduction at the or near by the place of ischemia or reperfusion is simultaneously or sequentially with the infusion of the said CD47 antibodies or fragments thereof.

10. The CD47 antibodies or fragments thereof for use in preventing or treating tissue injury in states of ischemia or reperfusion in an object according to any one of the preceding claims wherein the CD47 antibodies or fragments thereof are administered by catheter at the or near by the place of ischemia or reperfusion, in particular, the damaged organ, in a concentration sufficient to locally inhibit CD47 signaling for removing damaged cells by phagocytic machinery for improving reperfusion injury.

11. The CD47 antibodies or fragments thereof for use in preventing or treating tissue injury in states of ischemia or reperfusion in an object according to any one of the preceding claims wherein the administration or introduction is by a combination of a first administration of an initiate dosage in form of intraventricular injection of a bolus or intravenous injection of a bolus or by catheter to the coronary artery followed by continuous infusion.

12. The CD47 antibodies or fragments thereof for use in preventing or treating tissue injury in states of ischemia or reperfusion in an object according to any one of the preceding claims wherein the CD47 antibodies are an isolated chimeric or humanized antibody that specifically bind to human CD47, wherein said antibody comprises a heavy chain having each of the CDR sequences set forth in SEQ ID Nos. 3, 4, 5, 6, 7, 8, 9, 10, 11 and a light chain having each of the CDR sequences set forth in SEQ ID No. 12, 13, 14, 15, 16, 17, 18, 19, 20.

13. The CD47 antibodies or fragments thereof for use in preventing or treating tissue injury in states of ischemia or reperfusion in an object according to any one of claims 1 to 11 wherein the CD47 antibodies or fragments thereof are a monoclonal antibody or antigen binding fragment thereof which comprises a light chain variable region (LCVR) and a heavy chain variable region (HCVR), wherein said LCVR and said HCVR comprise, respectively, amino acid sequ3ences selected from among the following combinations of LCVRs and HCVRs:
wherein each one of the LCVR SEQ ID Nos 21 to 45 further comprises a constant domain having the amino acid sequence shown in SEQ ID No 46, and
wherein each one of the HCVR SEQ ID Nos 47 to 71 comprises a constant domain selected from among SEQ ID Nos 72, 73, 74, 75, 76.

14. The CD47 antibodies or fragments thereof for use in preventing or treating tissue injury in states of ischemia or reperfusion in an object according to claim 13 wherein the antibody or antigen binding fragment thereof comprises three light chain complementary determining regions (LCDRs 1 - 3) and three heavy chain complementary determining regions (HCDRs 1 - 3), wherein:
LCDR 1 comprises the amino acid sequence RSSQSLVHSNGNTYLH (SEQ ID NO: 77)
LCDR 2 comprises the amino acid sequence KVSYRFS (SEQ ID NO:78); and
LCDR 3 comprises the amino acid sequence SQNTHVPRT (SEQ ID NO:79) ;
HCDR1 comprises the amino acid sequence GYTFTNYYVF (SEQ ID NO:80);
HCDR 2 comprises the amino acid sequence DINPVNGDTNFNEKFKN (SEQ ID NO:81); and
HCDR 3 comprises the amino acid sequence GGYTMDY (SEQ ID NO:82).
